# EUROPEAN PATENT APPLICATION

(11) **EP 2 724 626 A1**
(43) Date of publication of application: **30.04.2014**
(21) Application number: 12803392.5
(22) Date of filing: 22.06.2012
(51) Int. Cl.: A23L 1/16, A23L 1/304

(54) **NOVEL NOODLE DOUGH AND NOODLE PROUCT USING SAME**

(30) Priority: 24.06.2011 JP 2011140774
(71) Applicant: Kyowa Chemical Industry Co., Ltd, Takamatsu-shi, Kagawa 761-0113 (JP)
(72) Inventor: ISHIHAMA, Satoru, Kita-gun Kagawa 761-0705 (JP)
(74) Representative: Raynor, Stuart Andrew
(86) International application number: PCT/JP2012/066005
(87) International publication number: WO 2012/176882

(57) **Abstract**

A noodle product having a high magnesium content.

The noodle product is produced by mixing concentrated bittern with wheat flour. That is, the noodle product of the present invention contains concentrated bittern, thereby providing a noodle product as a food with nutrient function claims which replenishes Mg as an essential mineral, does not induce high blood pressure and alleviates the morning sickness of a pregnant woman.

## Description

### TECHNICAL FIELD

The present invention relates to noodle dough having a high magnesium content produced by mixing wheat flour and/or buckwheat flour with concentrated bittern containing a large amount of magnesium and a noodle product thereof. That is, the noodle product of the present invention contains concentrated bittern having a high magnesium content in place of salt which is indispensable in the production of wheat noodles, thereby providing noodle dough containing a large amount of Mg as an essential mineral and a noodle product thereof.

### BACKGROUND ART

Noodles, for example, wheat noodles, fine noodles, buckwheat noodles, pasta, or noodles and Chinese noodles are eaten worldwide, and the process for producing noodle dough remains unchanged and basically comprises adding salt and water to wheat flour as the main material, kneading them together and molding the kneaded product into a linear form, etc.

It is known that egg albumen, a protein material, a polysaccharide thickener and a phosphoric acid salt are kneaded with noodle dough to improve the quality of noodles.

There are disclosed noodles containing large amounts of calcium, potassium, protein and food fibers which are produced by using only naturally-derived foodstuffs such as wheat flour, durum semolina flour, bean-curd refuse, vegetable seed fermented food, olive oil and saffron oil without using additives, water and eggs (patent document 1). It is disclosed that, by adding additive salt for noddle processing which comprises 70 to 95 wt% of sodium chloride, 20 to 2.5 wt% of magnesium chloride, 4 to 0 wt% of calcium chloride, 2 to 0 wt% of potassium chloride and 4 to 0 wt% of magnesium sulfate, the boiling time of the obtained noodles can be shortened, the texture and eating quality of the noodles become better, and extension by boiling is delayed (patent document 2).

However, all the noodles of the above patents contain salt as in the prior art and therefore, do not take into consideration high blood pressure, arterial sclerosis, cerebral embolism and kidney troubles. Although a process for producing noodles by adding bittern and not salt was invented thereafter, the obtained noodles contain a large amount of Na which induces high blood pressure (patent document 3). Bittern contains 10 to 40 g/L of sodium.

### Prior Art Documents

### Patent documents

Patent Document 1: JP-A 2008-178373
Patent Document 2: JP-A 11-32712
Patent Document 3: W02005/077206

### DISCLOSURE OF THE INVENTION

### Problems to Be Solved by the Invention

The present invention relates to noodle dough produced by mixing concentrated bittern with wheat flour and/or buckwheat flour and a noddle product thereof. That is, the noodle dough and noodle product of the present invention have a reduced content of conventional salt (NaCl) and contain concentrated bittern having a high magnesium content, thereby providing noodle dough and a noodle product thereof as a food with nutrient function claims which replenishes Mg as an essential mineral, do not induce metabolic syndrome, diabetes, high blood pressure and hyperlipidemia and can alleviate the morning sickness of a pregnant woman.
The inventors of the present invention found that, when this concentrated bittern having a high magnesium content is mixed, the obtained noodles taste mild and become firm. It is assumed that this is because the noodles containing concentrated bittern have little water absorption, that is, hardly absorbs water during boiling as compared with noodles which do not contain concentrated bittern.

The main reason that brine is used in noodle dough is that wheat flour is bound by the coagulation function of salt and the viscoelasticity and hardness of noodle dough are enhanced by salt. No problem occurs even when concentrated bittern is used in place of the salt of the present invention.

Although the above patent document 3 discloses a patent in which bittern is used, the concentrated bittern of the present invention may be naturally-concentrated bittern, bittern prepared by concentrating sea water or bittern artificially or bittern prepared by concentrating magnesium chloride which is the main component of bittern. The naturally-concentrated bittern is concentrated bittern produced in lakes naturally and a safe material having an acute toxicity value of 2,000 mg/kg or more in an acute oral toxicity test using mice.
The surface of a lake is covered with water in a rainy season but evaporated by powerful solar energy in a dry season to be changed into a hard thick salt layer. Below the salt layer, there exists thick water containing lots of minerals such as magnesium. When this thick lake water is bailed out into a pool on the ground and further concentrated with the heat of strong sunlight in a summer season, sodium is crystallized and precipitated before magnesium, thereby producing a magnesium-rich mineral liquid from which 99 % of sodium has been removed.

The main production area of this naturally-concentrated bittern is Lake Deborah in Australia. It is said that, in this area, strong west wind blows from the sea toward the inland, airborne droplets of the sea water of the Indian Ocean are carried to the inland by the west wind and accumulate in Lake Deborah which is far away from the area. The working of this great nature is still going on. Table 1 shows the components of naturally-concentrated bittern (trade name: Magneforce produced in Lake Deborah in Australia).

This naturally-concentrated bittern is completely different from bittern by-produced when salt is produced. Although the bittern has a high magnesium content, sodium still remains in some measure. The naturally-concentrated bittern used in the present invention has an extremely low sodium content and contains highly concentrated magnesium. Table 2 shows comparison between naturally-concentrated bittern (trade name: Magneforce, produced in Lake Deborah in Australia) and bittern produced along the shore of the Seto Inland Sea.

The process for concentrating bittern, sea water or magnesium chloride is, for example, a process comprising boiling down sea water or magnesium chloride to concentrate it, removing crystal matter from the concentrated liquid after it is cooled and repeating this. The content of sodium is reduced by removing the crystal matter.

**[Table 1]**

| Components contained in 100 mL of naturally-concentrated bittern | | |
|---|---|---|
| | Naturally-concentrated bittern | |
| Energy | 0 | Kcal |
| Protein | 0 | g |
| Fat | 0 | g |
| Carbohydrate | 0 | g |
| Magnesium | 10,300 | mg |
| Potassium | 633 | mg |
| Sodium | 584 | mg |
| Calcium | 4.6 | mg |
| Zinc | 47 | µg |
| Copper | 26 | µg |
| Manganese | 11 | µg |
| Silicon | 1.43 | ppm |
| Phosphorus | 0.26 | ppm |
| Molybdenum | 0.15 | ppm |
| Iron | 0.14 | ppm |
| Chromium | 2.7 | ppb |
| Selenium | 0.73 | ppb |

**[Table 2]**

| | components | | |
|---|---|---|---|
| Analytical items | Naturally-concentrated bittern sample A | Bittern produced in the Seto Inland Sea | analyzing method |
| Sodium | 0.19g/100g | 3.39g/100g | atomic absorption photometry |
| Magnesium | 7.98g/100g | 3.78g/100g | atomic absorption photometry |
| Specific gravity (20°C) | 1.313 | 1.24 | oscillating densitometry |
| pH | 5.8 | 7.54 | glass electrode method |

A large amount of magnesium can be ingested by using this concentrated bittern in the noodle dough of the present invention. When a large amount of magnesium is ingested, diabetes, hyperlipidemia and metabolic syndrome are not induced, and an increase in the amount of calcium contained in the cell of the blood vessel can be suppressed with the result that effects such as the suppression of the shrinkage of the blood vessel and the reduction of the blood pressure can be obtained. As for pregnant women, the amount of magnesium contained in the cell increases in the early stage of pregnancy and the amount of magnesium in serum is reduced, thereby making it possible to alleviate morning sickness by replenishing magnesium.

The noodle product in the present invention is a product which is produced by using wheat flour and/or buckwheat flour as the main raw material and molded into a long and thin form, generally, wheat noodles, fine noodles, buckwheat noodles, pasta (including spaghetti), Chinese noodles or what are called "noodles". These noodle products may be dry noodles which are produced by hardening wheat flour or buckwheat flour with water or boiled noodles which are boiled in hot water. Further, they may be dry noodles produced by drying boiled noodles again. There is no standard for the amount of the raw material of the noodle dough, and the amount of the raw material is determined according to the shape and type of the noodles, the quality and state of flour, and taste and texture preferences. In the noodle product of the present invention, the concentrated bittern to be mixed with wheat flour or buckwheat flour (to be referred to as "raw material flour" hereinafter) has a magnesium content (as Mg) of 6.0 wt% or more, preferably 7.0 to 10 wt% and a sodium content (as Na) of 1.0 wt% or less, preferably 0.5 wt% or less. The noodle dough of the present invention is produced by mixing a small amount of salt, water and concentrated bittern with the raw material flour and kneading them together. The concentrated bittern contains magnesium (Mg) in an amount of 30 to 100 mg, preferably 40 to 90 mg based on 100 g of the raw material flour. When the amount of magnesium is smaller than 30 mg, the effect of magnesium is not fully obtained and when the amount of magnesium is 100 mg or more, magnesium and gluten contained in the raw material flour are bounded to each other with the result that the noodle dough becomes hard. The amount of water is adjusted according to preferences and small when the noodle dough is made hard and large when the noodle dough is made soft. When the amount of water is too small, the noodle dough becomes dry and when the amount of water is too large, the noodle dough becomes too soft. In either case, it is difficult to mold the noodle dough.
The noodle product of the present invention is generally boiled in water or hot water to be used as food. When the noodle product of the present invention is boiled, part of magnesium and part of sodium are eluted into hot water but part of magnesium remains even in the boiled noodle product. When the noodle product is boiled, it absorbs water right after boiling so that its weight increases 2 to 3 times. The magnesium content (Mg content) of the noodle product after boiling is suitably 30 to 50 mg based on 100 g of the noodle product before boiling.

When the noodles are to be boiled, concentrated bittern may be added to boiling water.

The following examples are provided for the purpose of further illustrating the present invention but are in no way to be taken as limiting.

### EXAMPLE 1

The naturally-concentrated bittern "sample A" (trade name: Magneforce, produced in Lake Deborah in Australia) shown in Table 2 was used in an amount shown below to be kneaded, the obtained noodle dough was stretched to be molded into a 5 mm-diameter linear form, and 100 g of the noodles was boiled in boiling water for 10 minutes to obtain wheat noodles. The components of the wheat noodles before and after boiling were analyzed and shown in Table 3. Wheat noodles which did not contain the naturally-concentrated bittern "sample A" were produced as Comparative Example 1.

### (composition)

| | |
|---|---|
| Wheat flour (all-purpose flour) | 500 g |
| Salt | 5 g |
| Water | 225 g |
| Magneforce (sample A) | 10 g |

### (composition of Comparative Example 1)

| | |
|---|---|
| Wheat flour (all-purpose flour) | 500 g |
| Salt | 25 g |
| Water | 225 g |

**[Table 3]**

| | Example 1 | | Comparative Example 1 | |
|---|---|---|---|---|
| Before boiling | | | | |
| Energy | 330 | Kcal | 335 | Kcal |
| Water | 35.0 | g | 37.0 | g |
| Ash | 5.0 | g | 5.1 | g |
| Protein | 8.2 | g | 8.0 | g |
| Fat | 1.2 | g | 1.0 | g |
| Carbohydrate | 71.7 | g | 73.0 | g |
| Magnesium | 63 | mg | 18 | mg |
| Calcium | 19 | mg | 18 | mg |
| Potassium | 140 | mg | 130 | mg |
| Sodium | 1.7 | g | 1.9 | g |
| Chlorine | 3.2 | g | 6.0 | g |
| | | | | |

| After boiling | | | | |
|---|---|---|---|---|
| Energy | 139 | Kcal | 105 | Kcal |
| Water | 65.5 | g | 70 | g |
| Ash | 0.5 | g | 0.6 | g |
| Protein | 3.5 | g | 2.8 | g |
| Fat | 0.5 | g | 0.4 | g |
| Carbohydrate | 30.0 | g | 35.2 | g |
| Magnesium | 15 | mg | 6 | mg |
| Calcium | 8.4 | mg | 5.5 | mg |
| Potassium | 11 | mg | 3.8 | mg |
| Sodium | 0.019 | g | 0.020 | g |
| Chlorine | 0.3 | g | 0.7 | g |

The values of the components after boiling in Table 3 above are based on 100 g of the noodle product after boiling. The weights of the noodle products of Example 1 and Comparative Example 1 increased 2.24 times higher than those of the noodle products before boiling. Therefore, the noodles of Example 1 after boiling contained 33.6 mg (15 x 2.24) of magnesium based on 100 g of the noodle product before boiling.

### Example 2

The noodles obtained in Example 1 and Comparative Example 1 were kept at a temperature of 18°C and a humidity of 85 % for 99 hours and then dried at a temperature of 25 to 34°C and a humidity of 60 to 80 % in a cool air dry warehouse for 37.5 hours to obtain dry noodles. 100 g of the dry noodles was boiled in boiling water for 20 minutes. The analytical values of components before and after the dry noodles were boiled are shown in Table 4.
15 samplers tried the boiled noodles of Example 2 and Comparative Example 2 to check their textures. The results are shown in Table 5.

**[Table 4]**

| | Example 2 | | Comparative Example 2 | |
|---|---|---|---|---|
| Before boiling | | | | |
| Energy | 335 | Kcal | 337 | Kcal |
| Water | 14.0 | g | 11.9 | g |
| Ash | 3.6 | g | 5.2 | g |
| Protein | 8.6 | g | 8.3 | g |
| Fat | 1.0 | g | 1.1 | g |
| Carbohydrate | 72.8 | g | 73.5 | g |
| Magnesium | 72 | mg | 23 | mg |
| Calcium | 8.3 | mg | 18 | mg |
| Potassium | 110 | mg | 130 | mg |
| Sodium | 1.3 | g | 1.9 | g |
| Chlorine | 2.3 | g | 5.3 | g |
| | | | | |

| After boiling | | | | |
|---|---|---|---|---|
| Energy | 137 | Kcal | 110 | Kcal |
| Water | 66.1 | g | 72.8 | g |
| Ash | 0.3 | g | 0.2 | g |
| Protein | 3.7 | g | 2.8 | g |
| Fat | 0.5 | g | 0.3 | g |
| Carbohydrate | 29.4 | g | 23.9 | g |
| Magnesium | 17 | mg | 3.9 | mg |
| Calcium | 3.3 | mg | 6.1 | mg |
| Potassium | 7.8 | mg | 3.2 | mg |
| Sodium | 0.012 | g | 0.018 | g |
| Chlorine | 0.2 | g | 0.5 | g |

The values of the components after boiling shown in Table 4 above are based on 100 g of the noodle product after boiling. The weight of the noodle product after boiling increased 2.24 times higher than that of the noodle product before boiling. Therefore, the noodles of Example 2 after boiling contained 38 mg (17 x 2.24) of magnesium based on 100 g of the noodle product before boiling.

**[Table 5]**

| | The invented noodles of Example 2 are better | Cannot say which is better | The noodles of Comparative Example 2 are better |
|---|---|---|---|
| Taste | 9 | 3 | 3 |
| Texture | 13 | 0 | 2 |
| Firmness | 15 | 0 | 0 |
| Overall | 12 | 1 | 2 |

### Example 3

Macaroni was produced by using the naturally-concentrated bittern (sample A) shown in Table 1.

### (composition)

Macaroni was produced by kneading 500 g of wheat flour containing durum semolina flour, all-purpose flour and hard flour in a ratio of 1:0.5:0.5 with 200 g of water, 10 g of Magneforce and 10 g of olive oil.
As Comparative Example 3, macaroni was produced by kneading 500 g of wheat flour containing durum semolina flour, all-purpose flour and hard flour in a ratio of 1:0.5:0.5 with 200 g of water and 10 g of olive oil.

The magnesium (Mg) content of each of 100 g of the macaroni of Example 3 and 100 g of the macaroni of Comparative Example 3 was measured.

**[Table 6]**

| | Example 3 | Comparative Example 3 |
|---|---|---|
| Before boiling | 78 mg | 18 mg |
| After boiling | 19 mg | 2 mg |

## Claims

1. A noodle product made from wheat flour and/or buckwheat flour containing concentrated bittern.

2. The noodle product according to claim 1, wherein the concentrated bittern has a magnesium content of 6.0 wt% or more.

3. The noodle product according to claim 1, wherein the concentrated bittern has a sodium content of 1.0 wt% or less.

4. The noodle product according to claim 1, wherein the concentrated bittern contains magnesium in an amount of 60 to 100 mg based on 100 g of wheat flour and/or buckwheat flour.

5. The noodle product according to claim 1, wherein the magnesium content of the noodle product of claim 4 after boiling is 30 to 50 mg based on 100 g of the noodle product before boiling.

6. The noodle product according to claim 1 which alleviates the morning sickness of a pregnant woman.

7. The noodle product according to claim 1 which is a food with nutrient function claims.

8. The noodle product according to claim 1 which is wheat noodles, fine noodles, buckwheat noodles, pasta, Chinese noodles or noodles.

9. The noodle product according to claim 1 which is a dry noodle product.

10. The noodle product according to claim 1, wherein the concentrated bittern is naturally-concentrated bittern produced in Lake Deborah.
